# EUROPÄISCHE PATENTSCHRIFT

(11) **EP 0 862 485 B1**
(45) Veröffentlichungstag und Bekanntmachung des Hinweises auf die Patenterteilung: **05.09.2001**
(21) Anmeldenummer: 96909918.3
(22) Anmeldetag: 18.04.1996
(51) Int. Cl.: A61N 5/06

(54) **GERÄT ZUR PHOTODYNAMISCHEN BEHANDLUNG VON LEBEWESEN BZW. ORGANEN DERSELBEN**
APPARATUS FOR THE PHOTODYNAMIC TREATMENT OF LIVING BEINGS OR ORGANS THEREOF
APPAREIL DE TRAITEMENT PHOTODYNAMIQUE D'ETRES VIVANTS OU DE LEURS ORGANES

(30) Priorität: 24.11.1995 WO PCT/AT19/00030
(43) Veröffentlichungstag der Anmeldung: 09.09.1998
(73) Patentinhaber: NAGYPAL, Tibor, 1210 Wien (AT); Hofmann, Günther, 1190 Wien (AT)
(72) Erfinder: NAGYPAL, Tibor, 1210 Wien (AT); Hofmann, Günther, 1190 Wien (AT)
(74) Vertreter: Itze, Peter, Dipl.-Ing.
(86) Internationale Anmeldenummer: AT9600075
(87) Internationale Veröffentlichungsnummer: WO9719725

(56) Entgegenhaltungen:
- EP-A- 0 473 861
- EP-A- 0 627 243
- WO-A-91/08015
- WO-A-95/11059
- WO-A-95/26217
- DE-A- 2 943 117

## Beschreibung

Die Erfindung bezieht sich auf ein Anordnung zur photodynamischen Behandlung von Lebewesen bzw. Geweben und/oder Organen derselben, bei welcher eine hinsichtlich Intensität, Zeit und/oder Wellenlänge bzw. Spektralbänder steuerbare Lichtquelle, eine Schaltvorrichtung, eine Stromquelle und eine Einrichtung zur Ermittlung der Reaktion des behandelten Lebewesens, Gewebes bzw. Organs vorgesehen ist,

Aus der DE 2 943 117 A1 geht ein Gerät hervor, mit welchem an dem Patienten eine Lichttherapie durchgeführt wird, wobei der Patient mit Softlicht aus einer Breitspektralbandlichtquelle bestrahlt und die an der Oberfläche des Körpers auftretenden Reaktionen mittels einer Elektrodenanordnung ermittelt werden.

Aus EP 0 473 861 A1 geht ein Gerät hervor, bei welchem der Patient mit niedrigfrequenten, kohärenten elektromagnetischen Feldern behandelt wird. Es handelt sich dabei um ein Gerät für eine spezielle Lichttherapie, bei welcher die angeführten magnetischen Felder zur Anwendung gelangen, wobei die Reaktion der Körperoberfläche des Patienten gemessen wird.

Die EP 0 627 243 A1 offenbart ebenfalls ein Gerät zur Vornahme einer Lichttherapie, wobei die Leistung und die Zeitdauer der Bestrahlung durch die von der Körperoberfläche des Patienten reflektierte Lichtintensität gesteuert wird.

Bei den bekannten Geräten erfolgt die Behandlung dadurch, daß durch die Lichtquelle für eine empirisch bestimmte Zeit das Licht ohne eine gezielte Regelung in das Gewebe emittiert wird.

Die photodynamische Behandlung von Lebewesen bzw. Organen derselben beruht darauf, daß im Körper durch spezielle Farbstoffsubstanzen biophotochemische Effekte ausgelöst werden, die sich beispielsweise in krebsbefallenen Geweben mit einer 10 - 30fachen Konzentration, bezogen auf das gesunde Gewebe, anreichern. Dies kann dann dazu führen, daß in solchen krebsbefallenen Geweben in den Zellen Singuletsauerstoff freigesetzt wird, welcher die Krebszellen selektiv vernichtet. Es hat sich nun bei Versuchen gezeigt, daß sich während der Lichteinwirkung die optischen Gewebeeigenschaften und die Gewebe-Photonen-Interaktionen kontinuierlich in verschiedenen schmalen Spektralbandbereichen ändern. Es liegt eine Reihe von gewebeeigenen Farbstoffsubstanzen vor, die auf die in einem schmalen Spektralbandwellenlängenbereich auf treffenden Photonen reagieren, wodurch dann die biophotochemischen Vorgänge hervorgerufen werden. Der Therapieeffekt ist damit insgesamt abhängig von der Farbstoffkonzentration, den optischen Eigenschaften des kranken Gewebes, der angewandten Lichtintensität, der spektralen Zusammensetzung des Lichtes und dem Allgemeinzustand des Patienten, wobei neben den verschiedenen Wellenlängen des Lichtes auch die Lichtintensität eine erhebliche Rolle spielt.

Der Erfindung liegt die Aufgabe zugrunde, eine Anordnung der eingangs genannten Art zu schaffen, welche gezielt für die photodynamische Behandlung eines Lebewesens ausgelegt ist.

Erfindungsgemäß wird dies dadurch erreicht, daß zur Adaptierung der Anordnung für eine adaptive, über rückgemeldete Daten an den behandelten Patient individuell angepaßte, auf ein Zusammenwirken mit biophotochemische Effekte auslösenden Farbstoffen und auf Gewebe-Photonen-Interaktionen beruhende Behandlung von Lebewesen bzw. Geweben und/oder Organen in der Anordnung ein Rechner vorgesehen ist, der eine Expertendatenbank und eine Arbeitsdatenbank aufweist und mit einem Komparator versehen ist, wobei der Rechner mit der Lichtquelle und mit die Reaktion der krebsbefallenen Zellen des behandelten krebskranken Lebewesens bzw. des krebskranken Gewebes oder Organs, erfassenden Sensoren zur Erfassung von die Reaktionen dieser krebsbefallenen Zellen anzeigenden Daten, wie Sauerstoffkonzentration, Gewebeimpedanz, Volumen, Durchblutung, NADH- bzw. MPA-Konzentration, oder Temperatur, verbunden ist und wobei über ein adaptives Kontrollsystem durch den Komparator durch Vergleich der erhaltenen Sensordaten mit den in der Expertendatenbank gespeicherten Daten die ermittelten Vergleichsdaten ausgewertet und danach der Rechner die Lichtquelle in Abhängigkeit von der Auswertung der Vergleichsdaten regelt. Dadurch wird es möglich, die Strahlungsintensität und die Wellenlänge bzw. die Spektralstruktur den jeweiligen biophotochemischen Vorgängen des behandelten Organs therapiezielbezogen anzupassen, wobei auch ein zeitlich veränderliches Spektralmuster in das Gewebe emittiert werden kann.

Unter Spektralmuster ist dabei ein zeitveränderliches, spektralstrukturiertes Licht zu verstehen, welches aus einem oder mehreren Spektralbändern besteht. Das einfachste Spektralmuster kann mittels eines vierdimensionalen Vektors charakterisiert werden, bei welchem die Vektorkoordinaten die Energie des Bandes, die Bandbreite, die mittlere Bandwellenlänge und die Zeit sind. Diese vier Vektoren werden über einen entsprechenden Rechner der Lichtquelle vorgegeben, wobei der Rechner den individuellen Daten entsprechend, voreingestellt wird. Im Unterschied zu den bekannten Anordnungen wird mit Hilfe der Photosensibilisatoren der photodynamischen Therapie eine gezielte Reaktion eines bestimmten Organs, Gewebes od.dgl. hervorgerufen, wobei die therapiezielbezogenen Daten, die somit auf der Therapie beruhen, gemessen werden, u.zw. in dem Rechner, der sowohl die Daten verarbeitet als auch die Lichtquelle bezüglich der angeführten Daten steuert, wobei der Rechner auch mit einer oder mehreren Datenbanken versehen ist, um entsprechende Verarbeitungsschritte setzen zu können. Insbesondere werden mittels der Sensoren jene Daten erfaßt, die die Reaktionen der krebsbefallenen Zellen anzeigen. Es handelt sich dabei um jene physikalischen und/oder physiologisch chemischen Daten der Zellen, die sich aufgrund des Krebsbefalles während der Behandlung verändern, nämlich um Sauerstoffkonzentration, Gewebeimpedanz, Volumen, Durchblutung, NADH- bzw. MPA-Konzentration, oder Temperatur. Diese Daten sind auf Seite 10, Zeilen 12 - 26, der ursprünglich eingereichten Beschreibung angegeben. Weiters wurde im Anspruch noch angeführt, daß der Rechner die Lichtquelle zur Erzielung eines vorgegebenen Spektralmusters (Photonenenergiemuster), vorzugsweise einer Sequzenz von Mustern, regelt.

Da der Rechner mit einem Komparator zum Vergleich der von den Sensoren erfaßten Daten mit jenen der Expertendatenbank versehen ist und da bereits aufgrund klinischer Erfahrungen ermittelte Eckdaten im Rechner vorliegen, auf welche zur Behandlung bestimmter Krankheiten bereits auf klinisch erprobte Spektralmuster zurückgegriffen werden kann, erübrigt sich damit ein unter Umständen langwieriges Experimentieren. Die Sensoren stehen mit einer Arbeitsdatenbank in Verbindung, die die ermittelten Daten für den Komparator aufarbeitet, wobei die Arbeitsdatenbank an die Expertendatenbank angeschlossen ist, wodurch sofort ermittelt werden kann, ob das Gewebe auf das Spektralmuster in der erwünschten Weise anspricht. Auch ist es möglich, die Expertendatenbank im Hinblick auf die vom Gewebe zurückgemeldeten Daten zu korrigieren, um eine auf das jeweilige Individuum abgestellte optimale Wirkung des Gerätes zu erzielen.

Bei einer besonders bevorzugten Ausbildung können die Sensoren zur Erfassung von Daten des gesunden Gewebes ausgebildet und an eine Referenzdatenbank angeschlossen sein, wobei der Komparator zum Vergleich der von den Sensoren erfaßten Sollwerte des gesunden Gewebes mit den Istwerten des kranken Gewebes ausgebildet ist.

Der Lichtquelle kann ein Strahlkoppler zum gezielten Einbringen der Lichtstrahlen in das Gewebe vorgeschaltet sein, womit eine optimale Ausnützung der Lichtstrahlung erreicht ist. Dabei kann der Strahlkoppler ein Linsen- und/oder Spiegelsystem zum Ausrichten und/oder Selektieren der Lichtstrahlen aufweisen. Damit ist es möglich, die entsprechenden Spektralmuster gezielt auszubilden und die Strahlen entsprechend ausgerichtet in das Gewebe einzubringen. Zum gleichen Zweck kann zum Ausrichten und/oder Selektrieren der Lichtstrahlen in den Strahlengang ein Reflexionsprisma oder -kegel eingeschaltet sein.

Bei einer weiteren Ausführung des Strahlkopplers kann eine Lichtfaseroptik zum Ausrichten und/oder Selektieren der Lichtstrahlen vorgesehen sein. All das dient zu dem bereits oben angeführten Zweck der gezielten Ausbildung des Photonenenergiemusters. Weiters können die im Strahlkoppler vorgesehenen Einrichten zum Ausrichten und/oder Selektieren der Lichtstrahlen zur geometrischen Gestaltung der zu bestrahlenden Fläche zueinander verstellbar sein, was den Zweck hat, daß die Bestrahlung ausschließlich auf das kranke Gewebe ausgerichtet wird und gesundes Gewebe durch die Bestrahlung nicht erfaßt wird.

Um den Sensoren die vom Gewebe erzeugten Daten gezielt zuzuführen, können den Sensoren Sensorenkoppler zum gezielten Erfassen der Daten des zu behandelnden Gewebes vorgeschaltet sein. Dabei können die Sensorenkoppler den Strahlkopplern analog aufgebaut sein, um solcherart die Photonenmuster zu analysieren und entsprechend aufbereitet an den Rechner weiterzugeben. Dabei kann der Sensorkoppler zusätzlich zu den Linsen und/oder Spiegel und/oder Lichtfasersystemen noch Elektroden und/oder Meßsonden zur zusätzlichen Ermittlung physikalischer und/oder physiologisch-chemischer Meßdaten enthalten, da anhand dieser physikalischen und/oder physiologisch-chemischer Meßdaten die biologische Reaktion der Zellen beurteilt werden kann.

Wenn als Lichtquelle eine Breitbandlichtquelle vorgesehen ist, der mehrere Spektralbandfilter und/oder Intensitätsmodulen nachgeschaltet sind, kann jede Komponente gesondert steuerbar sein, um solcherart ein feineres Spektralmuster erzeugen zu können. Es kann aber auch eine Breitbandlichtquelle mit nachgeschaltetem Spektralbandfilter und/oder Intensitätsmodulen mit einem intensitätsgesteuerten Laser kombiniert sein, was eine kombinierte Behandlung von Geweben ermöglicht, falls dies als günstig eingestuft wird, wobei zusätzlich noch erreicht wird, daß eine hohe Universialität des Gerätes vorliegt. Damit das über die Strahlkoppler einzubringende Licht bereits entsprechend aufbereitet dem Strahlkoppler zugeleitet wird, kann den Spektralbandfiltern und/oder Intensitätsmodulen bzw. dem intensitätsgesteuerten Laser ein Lichtintegrator nachgeschaltet sein, wobei bei kombinierten Ausbildungen der Lichtintegrator für alle Einheiten gemeinsam ist. Dabei kann der Ausgang des Lichtintegrators direkt mit dem Eingang des Strahlkopplers verbunden sein.

Um eine gezielte Erfassung der Daten zu ermöglichen, können die Sensoren zur getrennten Erfassung der Spektralmuster und/oder elektrophysiologischer und/oder physiologisch-chemischer Daten ausgebildet sein, wodurch erreicht wird, daß jedes Signal getrennt erfaßt und auch in dieser Form verarbeitbar ist. Dabei kann der Sensor zur Ermittlung der Spektralmuster Monochromatoren und/oder, gegebenenfalls steuerbare, Filter und/oder Spektralanalysatoren aufweisen, wodurch eine genaue Analyse der von dem Gewebe rückgemeldeten spektralen Muster ermöglicht ist.

Für eine besonders genaue Bearbeitung der von den Sensoren ermittelten Daten, kann jedem der Sensoren ein auf die zu ermittelnden Daten abgestimmter Datenumwandler nachgeschaltet sein, der diese Daten dann entsprechend verarbeitet der Arbeitsdatenbank übermittelt.

In der Zeichnung sind Ausführungsbeispiele des Erfindungsgegenstandes in Form schematischer Blockdarstellungen der Komponenten wiedergegeben.

Fig. 1 zeigt ein herkömmliches Behandlungsgerät.

Fig. 2 gibt einen Bestrahlüngsteil des Behandlungsgerätes wieder, bei welchem über experimentell ermittelte Daten die Lichtquelle steuerbar ist.

Fig. 3 veranschaulicht schematisch eine erste Ausbildung eines Strahlkopplers mit einer Breitbandlichtquelle.

Fig. 4 gibt eine abgeänderte Ausbildung des Strahlkopplers für eine Breitbandlichtquelle wieder.

Fig. 5 zeigt einen Strahlkoppler mit Lichtleiterfasern, wobei für jedes Spektrum eine eigene Faser vorgesehen ist.

Fig. 6 gibt eine der Fig. 5 ähnliche Ausbildung wieder, wobei von der Lichtquelle nur ein Faserstrang ausgeht, der sich dann je nach Anzahl der Spektralbänder aufspaltet.

Fig. 7 zeigt den Aufbau der regelbaren Lichtquelle, welche eine Breitspektralband emittierende Lichtquelle aufweist.

Fig. 8 gibt eine der Fig. 7 ähnliche Ausbildung, jedoch für intensitätssteuerbare Laser, wieder.

Fig. 9 zeigt eine kombinierte Ausbildung, welche eine Breitspektralband emittierende Lichtquelle und mehrere intensitätssteuerbare Laser aufweist.

Fig. 10 zeigt eine Gesamtzusammenstellung des erfindungsgemäßen Gerätes, welches zusätzlich zum Bestrahlungsteil auch einen Sensorteil zur Ermittlung von Gewebsdaten aufweist.

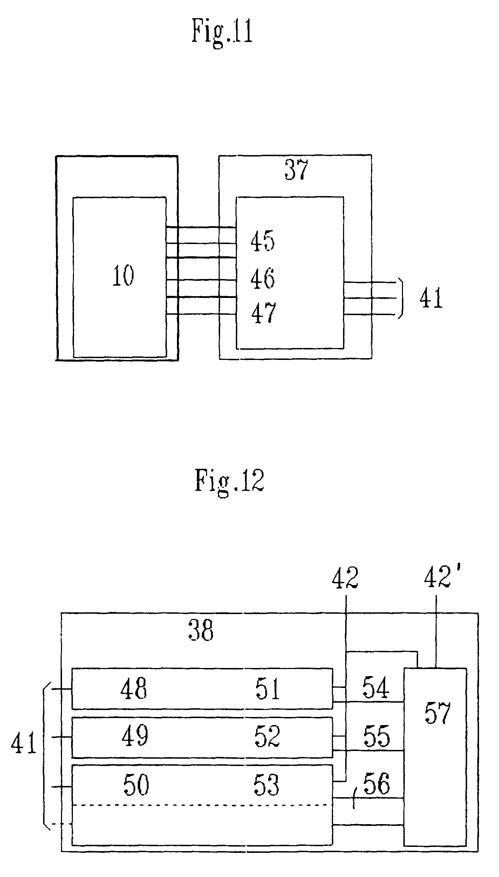

Fig. 11 veranschaulicht den Aufbau eines Sensorkopplers und die Verbindung zum Gewebe.

Fig. 12 gibt dann schematisch den Aufbau des Sensors als solchen wieder. Fig. 13 gibt den Spektralsensor in größerem Detail wieder.

Fig. 14 veranschaulicht einen Bestrahlüngsteil analog Fig.2 mit intensitätssteuerbaren Lasern, wobei die Steuerung mittels einer Expertendatenbank ohne Rückmeldung aus dem Gewebe erfolgt.

Fig. 15 zeigt eine der Fig. 14 analoge Ausbildung, welche erfindungsgemäß zusatzlich noch Sensorkoppler und Sensoren zur Ermittlung von durch die Bestrahlung geänderten Gewebsdaten besitzt.

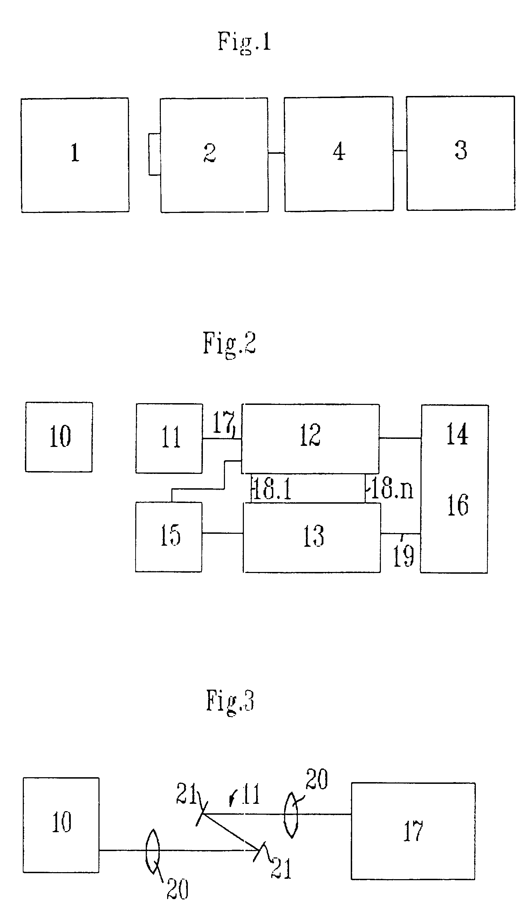

Bei der bekannten Ausbildung gemäß Fig. 1 wird das Gewebe 1 des Patienten mit einer Lichtquelle 2 bestrahlt, die von einer Stromversorgung 3 beaufschlagt ist. Diese Lichtquelle 2 ist dabei über eine Ein- und Ausschaltvorrichtung 4 schaltbar. Wie ersichtlich, wird die Lichtquelle in bezug auf das Gewebe des Patienten distanziert plaziert, sodaß eine eher unspezifische Strahlung auf das Gewebe eintrifft, wobei beachtliche Strahlungsverluste an die Umgebung nicht ausgeschlossen werden können.

Bei dem in Fig.2 wie dergegebenen Bestrahlüngsteil des erfindungsgemäßen Gerätes wird das kranke und/oder gesunde Gewebe 10 über einen Strahlkoppler 11 mit einer Lichtquelle 12 verbunden, deren Intensität und/oder Spektralmuster steuerbar ist. Für diese Steuerung ist eine Mustersteuerelektronik 13 vorgesehen, die über einen Rechner 14 angesteuert wird. Sowohl die Lichtquelle 12 als auch die Mustersteuerelektronik 13 und der Rechner 14 sind von einer Stromquelle 15 versorgt. Innerhalb des Rechners ist eine Expertendatenbank 16 vorgesehen, in welcher empirisch ermittelte Daten für die Behandlung des kranken Gewebes abgelegt sind.

Für den Betrieb der Vorrichtung wird der Strahlkoppler 11 so an das kranke Gewebe herangebracht, daß das vom Strahlkoppler bestrahlte Feld dem kranken Gewebe entspricht. Der Strahlkoppler empfängt die Lichtstrahlen bzw. das Spektralmuster über den Strahlenausgang 17 der Lichtquelle 12, welche über die Leitungen 18.1 bis 18.n, mit der Mustersteuerelektronik 13 verbunden ist. Die Anzahl der Leitungen 18 richtet sich nach der Anzahl der Bänder bzw. Spektralbereiche, mit welcher die Lichtquelle beaufschlagt ist. Die Mustersteuerelektronik bekommt die entsprechenden Steuerbefehle über den Steuersignalausgang 19 vom Rechner 14, der die entsprechenden Daten aus der Expertendatenbank entnimmt.

In den Fig. 3 - 6 sind verschiedene Varianten des Strahlkopplers 11 schematisch wiedergegeben, wobei Fig. 3 einen aus Linsen 20 und Umlenkspiegeln 21 aufgebauten Strahlkoppler wiedergibt. Durch entsprechende Wahl bzw. Anordnung der Linsen und entsprechendes Verschwenken der Spiegel 21 kann eine Änderung des vom Strahlkoppler ausgegebenen Musters erfolgen.

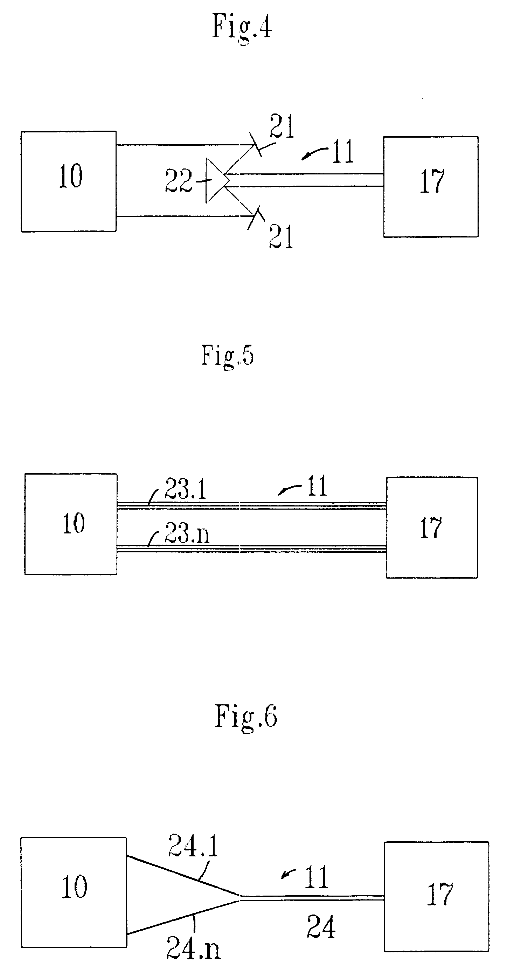

Gemäß Fig. 4 beaufschlagt der Strahlausgang 17 einen Strahlkoppler 11, bei welchem die Lichtstrahlen auf einen Brechungskegel oder ein Brechungsprisma 22 auftreffen, wobei die reflektierten Lichtstrahlen über Umlenkspiegel 21 auf das Gewebe 10 geleitet werden.

Gemäß Fig. 5 ist der Strahlkoppler als Lichtleitfaserbündel ausgebildet, wobei eine vorgegebene Anzahl von Lichtfasern 23, u.zw. die Fasern 23.1 bis 23.n besteht. Jede dieser Fasern übermittelt einen bestimmten Spektralbereich, sodaß die Gemeinsamkeit der Lichtfasern dann ein vorgegebenes Spektralmuster an das Gewebe 10 abgibt. Diese Ausführungsvariante kann gemäß Fig. 6 dadurch abgeändert werden, daß ein einziges Faserbündel 24 vom Strahlausgang 17 wegführt, welches am Weg in ein Faserbündel 24.1 bis 24.n aufgeteilt wird, wobei durch diese Aufteilung gleichfalls ein bestimmtes Spektralmuster erreichbar ist.

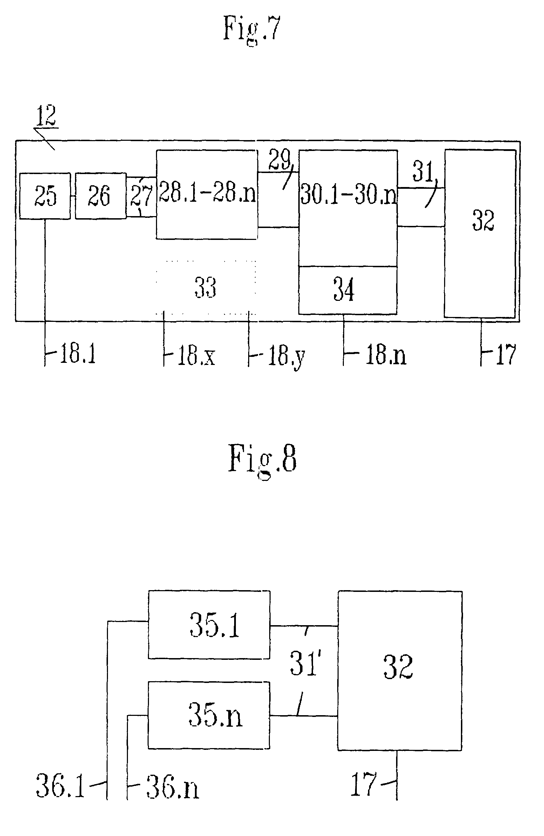

In Fig. 7 ist der Aufbau der Intensität und/oder spektralsteuerbaren Lichtquellen wiedergegeben, wobei diese von der Mustersteuerelektronik über die Leitungen 18.1 bis 18.n beaufschlagt ist. Die in Fig. 7 angeführten Leitungen 18.x und 18.y sind Leitungen der Gruppe 18.1 bis 18.n und führen ebenfalls von der Mustersteuerelektronik 13 zu dem jeweils notwendigen Teil der intensitäts- und/oder spektralsteuerbaren Lichtquelle 12. Diese Lichtquelle weist eine Breitspektralband emittierende Lichtquelle 25 auf, die mit einem Lichtverteiler und/oder Blende 26 verbunden ist. Das von dem Lichtverteiler und/oder Blende 26 ausgehende Licht wird über eine Leitung 27 am Spektralbandfilter 28.1 bis 28.n weitergeleitet und dort in ein entsprechendes Spektralbandmuster zerlegt. Dieses Spektralbandmuster wird dann über Leitungen 29 Intensitätsmodulatoren 30.1 bis 30.n, welche dann die intentitätsmodulierten Lichtstrahlen über die Leitung 31 einem Lichtintegrator 32 zuführen. Das in den Lichtintegrator eintretende Licht wird über den Strahlausgang 17 dem Strahlkoppler zugeleitet und von diesem dann an das Gewebe 10 abgestrahlt. Die Spektralbandfilter 28.1 bis 28.n sind über ein Spektralfilter 33 steuerbar, welches über die Leitungen 18.x bis 18.y von der Mustersteuerelektronik beaufschlagt ist. Die Intensitätsmodulatoren 30.1 bis 30.n sind über eine entsprechende Modulatorsteuerelektronik 34 steuerbar, welche über eine Leitung 18.n ebenfalls von der Mustersteuerelektronik 13 mit Daten versorgt wird. Wie schon in Fig. 2 angeführt, wird diese Mustersteuerelektronik 13 über einen Signalausgang 19 vom Rechner 14 mit Daten aus der Expertendatenbank 16 versorgt.

Gemäß Fig. 8 ist anstelle einer Breitspektralband emittierenden Lichtquelle 25 als Lichtquelle wenigstens ein intensitätssteuerbarer Laser 35.1 vorgesehen, wobei - wie aus Fig. 8 ersichtlich ist - eine unbestimmte Zahl bis 35.n vorhanden sein kann. Diese intensitätssteuerbaren Laser sind über Leitungen 36.1 bis 36.n mit Intensitätssteuereinrichtungen verbunden, über welche die Laser entsprechend beaufschlagbar sind. Das aus den intensitätssteuerbaren Lasern, die aufgrund der Laserausbildung bereits ein spezielles Spektralband aufweisen, austretende Licht wird über die Leitung 31' wieder dem Lichtintegrator 32 zugeführt, der dann über den Strahlausgang 17 wieder den Strahlkoppler 11 beaufschlagt. Durch die einzelne Steuerbarkeit der intensitätssteuerbaren Laser 35.1 bis 35.n kann jedes der Spektralbänder hinsichtlich der Intensität und/oder Zeit gesondert gesteuert werden, was auf einfachere Weise erfolgen kann als bei der Ausbildung gemäß Fig. 7, wo dann die Intensitätsmodulatoren über eine spezielle Modulatursteuerelektronik 34 gesteuert werden muß, wobei auch die Spektralfiltersteuerung 33 entsprechend beaufschlagt werden muß, u.zw. im Zusammenwirken mit der Modulatorsteuerelektronik 34.

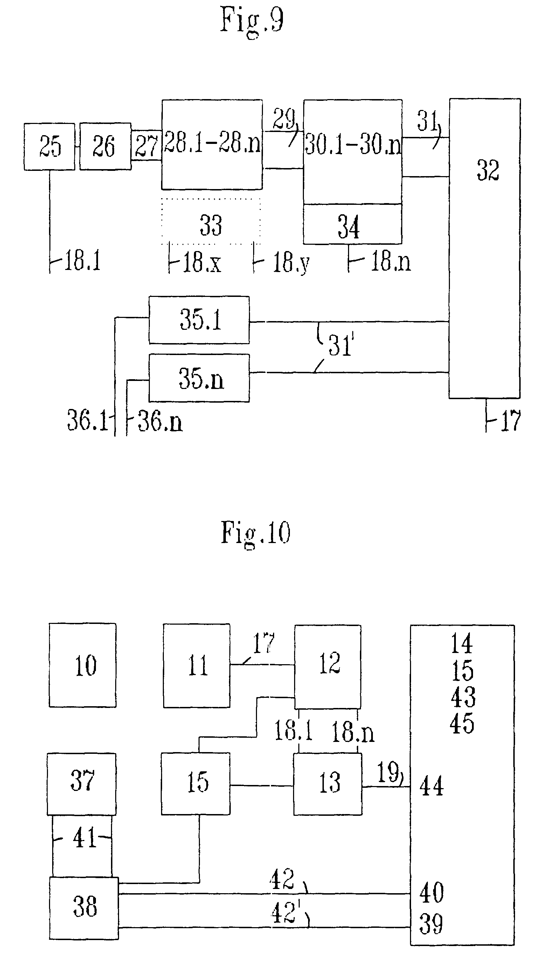

Die in der Fig. 9 wiedergegebene Ausführungsvariante ist eine Kombination der Ausführungen der Fig. 7 und 8, wobei die gleichen Bezugszeichen wie in den Fig. 7 und 8 verwendet sind. Dies gibt die Möglichkeit, eine noch genauere Regelung der Bestrahlung zu erreichen, da die Laserstrahlen den von der Breitspektralband emittierenden Lichtquelle kommenden Strahlen als Ergänzung beigeordnet werden können, sodaß hier ganz schmale Spektralbereiche verstärkt werden können, um so die gewünschte Bestrahlung zu erzielen.

Die Ausführungsvariante gemäß Fig. 10 weist den anhand Fig.2 beschriebenen Bestrahlungsteil auf, jedoch ist zusätzlich noch ein Datenerfassungsteil vorgesehen, welcher aus einem Sensorkoppler 37, einem Sensor 38, einem Sensordateneingang 39 und einer Sensorsteuerung 40 besteht. Der Sensorkoppler 37 ist über entsprechende Leitungen 41 mit den Sensoren 38 verbunden, wobei zur Sensorsteuerung ein entsprechender Sensorsteuerkanal 42 und ein Sensordatenkanal 42' führt. Innerhalb des Rechners 14 ist zusätzlich zu der Sensorsteuerung 40 und dem Sensordateneingang 39 noch eine Arbeitsdatenbank 43, ein Komparator 44 und ein adaptives Kontrollsystem 45 vorgesehen.

Bei dieser Ausführung wird über den Strahlkoppler von der Lichtquelle 12 kommendes Licht in krankes und/oder gesundes Gewebe entsprechend den Werten der Expertendatenbank 16 über den Rechner 14 abgegeben. Gleichzeitig wird über den Sensorkoppler 37, die Leitung 41 und den Sensor 38 die Reaktion des Gewebes auf das abgegebene Licht ermittelt, wobei die entsprechenden Sensordaten über den Sensordatenkanal 42', den Sensordateneingang 39, dem Rechner 14 zugeführt werden. Dieser Rechner verarbeitet dann die Daten des Sensordateneinganges über die Arbeitsdatenbank 43 und den Komparator 44, welcher die über den Sensordateneingang 39 ermittelten Daten mit jenen der Expertendatenbank 16 vergleicht. Über das adaptive Kontrollsystem 45 werden dann diese Vergleichsdaten ausgewertet und ermittelt, ob das Gewebe Werte gibt, die jenen der Expertendatenbank näher liegen oder weiter entfernt sind. Dadurch wird über das adaptive Kontrollsystem die Reaktionsrichtung ermittelt und je nach ermitteltem Wert und Trend dann die Sensorsteuerung 40 und die Mustersteuerelektronik entsprechend spezielle Steuerung 51, 52 und 53 vorgeschaltet, welche über Leitungen 54, 55, 56 von der Sensordatensteuerung 57 beaufschlagt sind, die über die Sensorsteuerung 42 beaufschlagt sind. Die Sensordaten werden vom Datenausgang der Spektralsteuerung 51 der Parametersteuerung 52 oder 53 abgeleitet und ebenfalls der Sensordatensteuerung 57 zugegeben, welche die Sensordaten dann über die Leitung 43 dem Sensordateneingang 39 des Rechners 14 zuleiten.

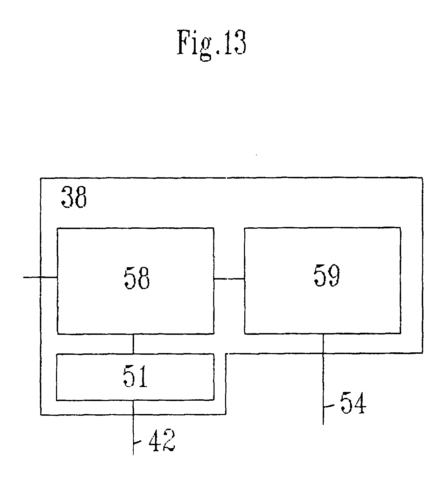

Bei den in Fig. 13 wiedergegebenen Details des Spektralsensors 48 sind Monochromatoren und/oder Filter bzw. steuerbare Filter und/oder Spektralanalysatoren 58 vorgesehen, die über die Spektralsensorsteuerung 51 beaufschlagt sind, welche ihrerseits wieder über die Leitung 42 von der Sensorsteuerung 40 die Befehle erhält. Die von den Monochromatoren und/oder Filtern und/oder steuerbaren Filtern bzw. Spektralanalysatoren 58 ermittelten Daten werden über einen photoelektrischen Umwandler 59 und den entsprechenden Datenausgang 54 der Sensordatensteuerung 57 zugeleitet.

Die Ausbildung gemäß Fig. 10 kann dabei so gesteuert sein, daß die über die Sensorkoppler 37, die Sensoren 38, den Sensordatenkanal 42' und dem Rechner 14 zugeleitete Daten jene sind, die von einem gesunden Gewebsteil abgelesen werden. Diese Daten werden dann der Expertendatenbank 16 als sogenannte Sollwerte eingegeben und das Gewebe über die Lichtquelle 12 und den Strahlkoppler 10 bestrahlt, wobei die Reaktion des Gewebes wieder über Sensoren ermittelt wird. Es werden dann die über das gesunde Gewebe ermittelten Solldaten der Expertendatenbank mit den von den Sensoren des kranken Gewebes nach der Bestrahlung ermittelten Daten im Komparator 44 verglichen und über die Arbeitsdatenbank ausgewertet, wobei dann das adaptive Kontrollsystem 45 wieder die Richtung der Reaktion auswertet.

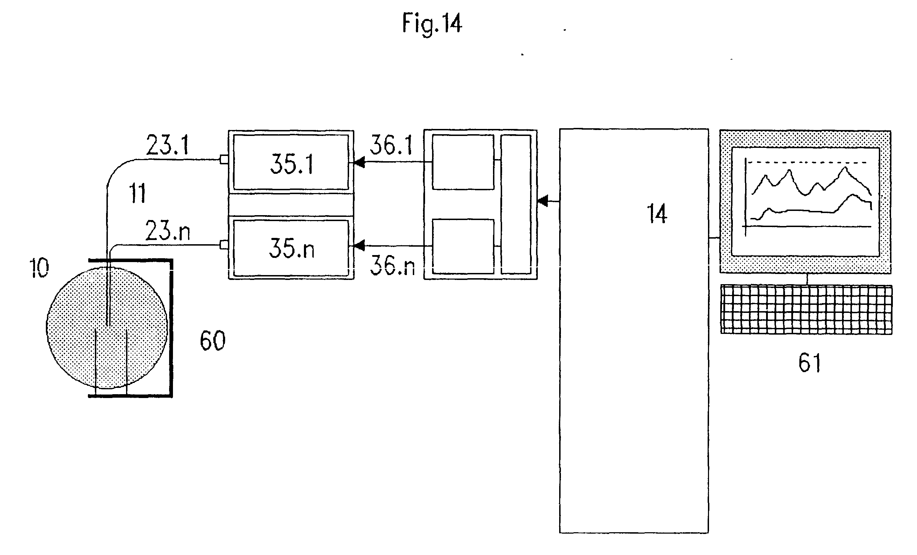

Fig. 14 zeigt eine der Fig. 2 analoge Bestrahlungseinrichtung, u.zw. ein mit intensitätssteuerbaren Lasern 35.1 bis 35.n ausgerüstetes Gerät, wobei die Laser über Leitungen 36.1 bis 36.n vom Rechner 14 über DA-Konverter und einen Adressen- und Datenseparator beaufschlagt sind. Mit 60 ist ein Halter für die Strahlkoppler zum Ansetzen an das spezielle Steuerung 51, 52 und 53 vorgeschaltet, welche über Leitungen 54, 55, 56 von der Sensordatensteuerung 57 beaufschlagt sind, die über die Sensorsteuerung 42 beaufschlagt sind. Die Sensordaten werden vom Datenausgang der Spektralsteuerung 51 der Parametersteuerung 52 oder 53 abgeleitet und ebenfalls der Sensordatensteuerung 57 zugegeben, welche die Sensordaten dann über die Leitung 43 dem Sensordateneingang 39 des Rechners 14 zuleiten.

Bei den in Fig. 13 wiedergegebenen Details des Spektralsensors 48 sind Monochromatoren und/oder Filter bzw. steuerbare Filter und/oder Spektralanalysatoren 58 vorgesehen, die über die Spektralsensorsteuerung 51 beaufschlagt sind, welche ihrerseits wieder über die Leitung 42 von der Sensorsteuerung 40 die Befehle erhält. Die von den Monochromatoren und/oder Filtern und/oder steuerbaren Filtern bzw. Spektralanalysatoren 58 ermittelten Daten werden über einen photoelektrischen Umwandler 59 und den entsprechenden Datenausgang 54 der Sensordatensteuerung 57 zugeleitet.

Die Ausbildung gemäß Fig. 10 kann dabei so gesteuert sein, daß die über die Sensorkoppler 37, die Sensoren 38, den Sensordatenkanal 42' und dem Rechner 14 zugeleitete Daten jene sind, die von einem gesunden Gewebsteil abgelesen werden. Diese Daten werden dann der Expertendatenbank 16 als sogenannte Sollwerte eingegeben und das Gewebe über die Lichtquelle 12 und den Strahlkoppler 10 bestrahlt, wobei die Reaktion des Gewebes wieder über Sensoren ermittelt wird. Es werden dann die über das gesunde Gewebe ermittelten Solldaten der Expertendatenbank mit den von den Sensoren des kranken Gewebes nach der Bestrahlung ermittelten Daten im Komparator 44 verglichen und über die Arbeitsdatenbank ausgewertet, wobei dann das adaptive Kontrollsystem 45 wieder die Richtung der Reaktion auswertet.

Die in Fig. 14 wiedergegebene Ausführungsvariante zeigt eine der Fig. 2 analoge Bestrahlungseinrichtung, u.zw. ein mit intensitätssteuerbaren Lasern 35.1 bis 35.n ausgerüstetes Gerät, wobei die Laser über Leitungen 36.1 bis 36.n vom Rechner 14 über DA-Konverter und einen Adressen- und Datenseparator beaufschlagt sind. Mit 60 ist ein Halter für die Strahlkoppler zum Ansetzen an das Gewebe 10 bezeichnet. Die Eingabe- und Überwachungseinheit ist mit 61 generell angedeutet.

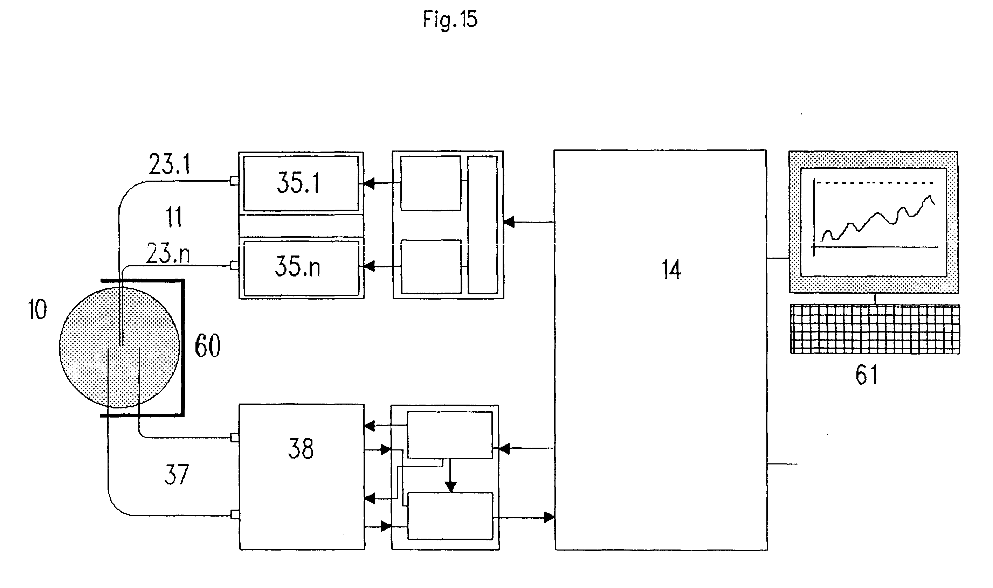

Das in Fig. 15 wiedergegebene erfindungsgemäße Gerät entspricht dem in Fig. 10 dargestellten Gerät, wobei die Bestrahlungseinrichtung wieder durch intensitätssteuerbare Laser gebildet sind. Der Sensor 38 weist dabei ein steuerbares Spektralphotometer und eine pH-Sonde auf, welche jeweils einen D/A-Konverter aufweisen und über einen Adress- und Datenselektor mit dem Rechner verbunden sind. Der Bestrahlungsteil der Anlage entspricht jenem der Fig. 14.

## Patentansprüche

1. Anordnung zur photodynarnischen Behandlung von Lebewesen bzw. Geweben und/oder Organen derselben, bei welcher eine hinsichtlich Intensität, Zeit und/oder Wellenlänge bzw. Spektralbänder steuerbare Lichtquelle, eine Schaltvorrichtung, eine Stromquelle und eine Einrichtung zur Ermittlung der Reaktion des behandelten Lebewesens, Gewebes bzw. Organs vorgesehen ist, **dadurch gekennzeichnet**, daß zur Adaptierung der Anordnung für eine adaptive, über rückgemeldete Daten an den behandelten Patient individuell angepaßte, auf ein Zusammenwirken mit biophotochemische Effekte auslösenden Farbstoffen und auf Gewebe-Photonen-Interaktionen beruhende Behandlung von Lebewesen bzw. Geweben und/oder Organen in der Anordnung ein Rechner (14) vorgesehen ist, der eine Expertendatenbank (16) und eine Arbeitsdatenbank (43) aufweist und mit einem Komparator (44) versehen ist, wobei der Rechner mit der Lichtquelle (12) und mit die Reaktion der krebsbefallenen Zellen des behandelten krebskranken Lebewesens bzw. des krebskranken Gewebes oder Organs, erfassenden Sensoren (38) zur Erfassung von die Reaktionen dieser krebsbefallenen Zellen anzeigenden Daten, wie Sauerstoffkonzentration, Gewebeimpedanz, Volumen, Durchblutung, NADH- bzw. MPA-Konzentration, oder Temperatur, verbunden ist und wobei über ein adaptives Kontrollsystem (45) durch den Komparator (44) durch Vergleich der erhaltenen Sensordaten mit den in der Expertendatenbank gespeicherten Daten die ermittelten Vergleichsdaten ausgewertet und danach der Rechner (14) die Lichtquelle (12) in Abhängigkeit von der Auswertung der Vergleichsdaten regelt.

2. Anordnung nach Anspruch 1, **dadurch gekennzeichnet**, daß die Sensoren (38) zur Erfassung von Daten des gesunden Gewebes ausgebildet und an eine Referenzdatenbank angeschlossen sind, wobei der Komparator (44) zum Vergleich der von den Sensoren (38) erfaßten Sollwerten des gesunden Gewebes mit den Istwerten des kranken Gewebes ausgebildet ist.

3. Anordnung nach einem der Ansprüche 1 oder 2, **dadurch gekennzeichnet**, daß der Lichtquelle (12) ein Strahlkoppler (11) zum gezielten Einbringen der Lichtstrahlung in das Gewebe vorgeschaltet ist.

4. Anordnung nach Anspruch 3, **dadurch gekennzeichnet**, daß der Strahlkoppler (11) ein Linsen (20) und/oder Spiegelsystem (21) zum Ausrichten und/oder Selektieren der Lichtstrahlen aufweist.

5. Anordnung nach Anspruch 4, **dadurch gekennzeichnet**, daß im Strahlkoppler (11) zum Ausrichten und/oder Selektieren der Lichtstrahlen in den Strahlgang ein Reflexionsprisma oder -kegel (22) eingeschaltet ist.

6. Anordnung nach Anspruch 3, **dadurch gekennzeichnet**, daß der Strahlkoppler (11) eine Lichtleiterfaseroptik (23.1-23.n,24,24.1-24.n) zum Ausrichten und/oder Selektieren der Lichtstrahlen aufweist.

7. Anordnung nach einem der Ansprüche 3 bis 6, **dadurch gekennzeichnet**, daß die im Strahlkoppler (11) vorgesehenen Einrichtungen zum Ausrichten und/oder Selektieren der Lichtstrahlen zur geometrischen Gestaltung der zu bestrahlenden Fläche zueinander verstellbar sind.

8. Anordnung nach einem der Ansprüche 1 bis 7, **dadurch gekennzeichnet**, daß den Sensoren (38) Sensorenkoppler (37) zum gezielten Erfassung der Daten des zu behandelnden Gewebes vorgeschaltet sind.

9. Anordnung nach Anspruch 8, **dadurch gekennzeichnet**, daß die Sensorkoppler (37) den Strahlkopplern (11) analog aufgebaut sind.

10. Anordnung nach Anspruch 8 oder 9, **dadurch gekennzeichnet**, daß der Sensorkoppler (37) zusätzlich zu den Linsen- und/oder Spiegel- und/oder Lichtfasersystemen (45) noch Elektroden (46) und/oder Meßsonden (47) zur zusätzlichen Ermittlung physikalischer und/oder physiologisch-chemischer Meßdaten enthält.

11. Anordnung nach einem der Ansprüche 1 bis 10, **dadurch gekennzeichnet**, daß bei mehreren einer Breitbandlichtquelle (25) nachgeschalteten Spektralbandfiltern (28.1-28.n) und/oder Intensitätsmodulen (30.1-30.n) jede einzelne Komponente gesondert steuerbar ist.

12. Anordnung nach einem der Ansprüche 1 bis 11, **dadurch gekennzeichnet**, daß eine Breitbandlichtquelle (25) mit nachgeschalteten Spektralbandfiltern (28.1-28.n) und/oder Intensitätsmodulen (30.1-30.n) mit einem oder mehreren intensitätsgesteuerten Laser(n) (35.1-35.n) kombiniert ist.

13. Anordnung nach Anspruch 11 oder 12, **dadurch gekennzeichnet**, daß den Spektralbandfiltern (28.1-28.n) und/oder Intensitätsmodulen (30.1-30.n) bzw. dem intensitätsgesteuerten Laser(n) (35.1-35.n) ein Lichtintegrator (32) nachgeschaltet ist, wobei bei kombinierten Ausbildungen der Lichtintegrator (32) für alle Einheiten gemeinsam ist.

14. Anordnung nach Anspruch 13, **dadurch gekennzeichnet**, daß der Ausgang (17) des Lichtintegrators direkt mit dem Eingang (17) des Strahlkopplers (11) verbunden ist.

15. Anordnung nach einem der Ansprüche 1 bis 14, **dadurch gekennzeichnet**, daß die Sensoren (38) zur getrennten Erfassung der Spektralmuster und/oder elektrophysiologischer und /oder physiologisch-chemischer Daten ausgebildet ist.

16. Anordnung nach Anspruch 15, **dadurch gekennzeichnet**, daß der Sensor (38) zur Ermittlung der Spektralmuster Monochromatoren und/oder, gegebenenfalls steuerbaren, Filtern und/oder Spektralanalysatoren (58) aufweist.

17. Anordnung nach Anspruch 15 oder 16, **dadurch gekennzeichnet**, daß jedem der Sensoren (38) ein auf die zu ermittelnden Daten abgestimmter Datenwandler nachgeschaltet ist.

## Claims

1. Arrangement for the photodynamic treatment of living beings or tissues and/or organs thereof, which comprises a light source which is adjustable in intensity, time and/or wavelength or bands of the spectrum, a switching device, a current source and means for detecting the reaction of the living being, tissue or organ being treated, **characterised in that**,for the purpose of adapting the apparatus for an adaptive treatment of living beings or tissues and/or organs which is individually tailored to the patient being treated by means of feedback data, based on interaction with dyes which trigger biophotochemical effects and on tissue/photon interactions, a computer (14) is provided in the apparatus, which comprises an expert data bank (16) and a working data bank (43) and is provided with a comparator (44), the computer being connected to the light source (12) and to sensors (38) which detect the reaction of the cancerous cells of the living being suffering from cancer who is being treated or the cancerous tissue or organ, for detecting data which indicate the reactions of these cancerous cells, such as oxygen concentration, tissue impedance, volume, bloodflow, NADH or MPA concentration, or temperature, and wherein the comparative data obtained are evaluated, via an adaptive monitoring system (45), by the comparator (44) by comparing the sensor data obtained with the data stored in the expert data bank, and then the computer (14) regulates the light source (12) as a function of the evaluation of the comparative data.

2. Arrangement according to claim 1, **characterised in that** the sensors (38) are designed to detect data from healthy tissue and are connected to a reference data bank, while the comparator (44) is designed to compare the nominal values of healthy tissue detected by the sensors (38) with the actual values of the diseased tissue.

3. Arrangement according to claim 1 or 2, **characterised in that** a beam coupler (11) is provided in advance of the light source (12) for the controlled introduction of light radiation into the tissue.

4. Arrangement according to claim 3, **characterised in that** the beam coupler (11) comprises a system of lenses (20) and/or mirrors (21) for directing and/or selecting the light beams.

5. Arrangement according to claim 4, **characterised in that** in the beam coupler (11) a reflection prism or cone (22) is inserted in the path of the beam for directing and/or selecting the light beams.

6. Arrangement according to claim 3, **characterised in that** the beam coupler (11) has light conducting fibre optics (23.1-23.n, 24, 24.1-24.n) for directing and/or selecting the light beams.

7. Arrangement according to one of claims 3 to 6, **characterised in that** the means provided in the beam coupler (11) for directing and/or selecting the light beams are adjustable to adapt to the geometric configuration of the area which is to be irradiated.

8. Arrangement according to one of claims 1 to 7, **characterised in that** sensor couplers (37) are provided upstream of the sensors (38) for the controlled detection of the data from the tissue which is to be treated.

9. Arrangement according to claim 8, **characterised in that** the sensor couplers (37) are constructed analogously to the beam couplers (11).

10. Arrangement according to claim 8 or 9, **characterised in that** the sensor coupler (37) contains, in addition to the lens and/or mirror and/or optical fibre systems (45), electrodes (46) and/or measuring probes (47) for additionally detecting physical and/or physiological/chemical measured data.

11. Arrangement according to one of claims 1 to 10, **characterised in that**, in the event of a plurality of spectral band filters (28.1-28.n) and/or intensity modules (30.1-30.n) provided downstream of a wideband light source (25), each individual component can be controlled separately.

12. Arrangement according to one of claims 1 to 11, **characterised in that** a wideband light source (25) with spectral band filters (28.1-28.n) and/or intensity modules (30.1-30.n) provided downstream thereof is combined with one or more variable-intensity laser(s) (35.1-35.n).

13. Arrangement according to claim 11 or 12, **characterised in that** a light integrator (32) is provided downstream of the spectral band filters (28.1-28.n) and/or intensity modules (30.1-30.n) or variable-intensity laser(s) (35.1-35.n), while in combined constructions the light integrator (32) is common to all the units.

14. Arrangement according to claim 13, **characterised in that** the output (17) from the light integrator is directly connected to the input (17) of the beam coupler (11) .

15. Arrangement according to one of claims 1 to 14, **characterised in that** the sensors (38) are designed to detect the spectral patterns and/or electrophysiological and/or physiological/chemical data separately.

16. Arrangement-according to claim 15, **characterised in that** the sensor (38) has monochromators and/or optionally controllable filters and/or spectral analysers (58) for detecting the spectral patterns.

17. Arrangement according to claim 15 or 16, **characterised in that** a data converter adapted to the data which are to be measured is provided downstream of each of the sensors (38).

## Revendications

1. Dispositif de traitement photodynamique d'êtres vivants ou de tissus et/ou d'organes d'êtres vivants, dans lequel il est prévu une source de lumière, dont l'intensité, la durée de fonctionnement et/ou la longueur d'onde ou les bandes spectrales peuvent être commandées, un dispositif de commutation, une source de courant et un dispositif pour déterminer la réaction de l'être vivant, du tissu ou de l'organe traité, **caractérisé en ce que** pour l'adaptation du dispositif pour un traitement adaptatif d'êtres vivants ou de tissus et/ou d'organes, qui est adapté individuellement au patient traité par l'intermédiaire de données transmises en retour et est basé sur une coopération avec des colorants déclenchant des effets biophotochimiques et sur des interactions tissu-photons, il est prévu dans le dispositif un calculateur (14), qui comporte une banque de données d'expert (16) et une banque de données de travail (43), et est équipé d'un comparateur (44), le calculateur étant relié à la source de lumière (12) et à des capteurs (38) détectant la réaction des cellules cancéreuses de l'être vivant traité atteint d'un cancer ou du tissu ou de l'organe atteint d'un cancer et servant à détecter des données qui indiquent la réaction de ces cellules atteintes d'un cancer, telle que la concentration en oxygène, l'impédance du tissu, le volume, le débit sanguin, la concentration de NADH ou de MPA, ou la température, et les données de comparaison déterminées étant évaluées, au moyen d'un système de contrôle adaptatif (45) par le comparateur (44) par comparaison des données fournies par le capteur aux données mémorisées dans la banque de données d'expert, et le calculateur (14) réglant ensuite la source de lumière (12) en fonction d'une évaluation des données de comparaison.

2. Dispositif selon la revendication 1, **caractérisé en ce que** les capteurs (38) servant à détecter des données du tissu sain sont formés et raccordés à une banque de données de référence, le comparateur (44) étant agencé de manière à comparer les valeurs de consigne, détectées par les capteurs (38), du tissu sain aux valeurs réelles du tissu malade.

3. Dispositif selon l'une des revendications 1 ou 2, **caractérisé en ce qu**'un coupleur de faisceau (11) servant à introduire de façon ciblée le rayon lumineux dans le tissu est branché en amont de la source de lumière (12).

4. Dispositif selon la revendication 3, **caractérisé en ce que** le coupleur de faisceau (11) comporte un système de lentilles (20) et/ou un système de miroirs (21) pour orienter et/ou sélectionner les faisceaux de lumière.

5. Dispositif selon la revendication 4, **caractérisé en ce qu**'un prisme ou un cône à réflexion (22) est inséré dans le coupleur de faisceau (11) pour orienter et/ou sélectionner les faisceaux de lumière dans le trajet du faisceau.

6. Dispositif selon la revendication 3, **caractérisé en ce que** le coupleur de faisceau (11) comporte un système optique à fibres formant guide de lumière (23.1-23.n, 24, 24.1-24.n) pour orienter et/ou sélectionner les faisceaux de lumière.

7. Dispositif selon l'une des revendications 3 à 6, **caractérisé en ce que** les dispositifs prévus dans le coupleur de faisceau (11) sont réglables l'un par rapport à l'autre de manière à orienter et/ou sélectionner les faisceaux de lumière pour réaliser la conformation géométrique de la surface à irradier.

8. Dispositif selon l'une des revendications 1 à 7, **caractérisé en ce que** des coupleurs (37) de capteurs sont branchés en amont: des capteurs (38) pour la détection ciblée des données du tissu à traiter.

9. Dispositif selon la revendication 8, **caractérisé en ce que** les coupleurs (37) des capteurs sont agencés d'une manière analogue aux coupleurs de faisceau (11).

10. Dispositif selon la revendication 8 ou 9, **caractérisé en ce que** le coupleur (37) de capteurs contient en plus des systèmes à lentilles et/ou des systèmes à miroirs et/ou des systèmes à fibre optique (45), également des électrodes (46) et/ou des sondes de mesure (47) pour la détermination supplémentaire de données de mesure physique et/ou physiologico-chimique.

11. Dispositif selon l'une des revendications 1 à 10, **caractérisé en ce que** dans le cas de plusieurs filtres de bande spectrale (28.1-28.n) et/ou des modules d'intensité (33.1-33.n), qui sont branchés en aval d'une source de lumière à large bande (25), chaque composant individuel peut être commandé séparément.

12. Dispositif selon l'une des revendications 1 à 11, **caractérisé en ce qu**'une source de lumière à large bande (25) équipée de filtres de bande spectrale (28.1-28.n) et/ou de modules d'intensités (30.1-30.n), branchés en aval, est combinée à un ou plusieurs lasers (35.1-35.n) dont l'intensité est commandée.

13. Dispositif selon la revendication 11 ou 12, **caractérisé en ce qu**'un intégrateur de lumière (32) est branché en aval des filtres de bande spectrale (28.1-28.n) et/ou des modules d'intensités (30.1-30.n) ou du ou des lasers (35.1-35.n), dont l'intensité est commandée, l'intégrateur de lumière (32) étant commun à toutes les unités, dans le cas de réalisations combinées.

14. Dispositif selon la revendication 13, **caractérisé en ce que** la sortie (17) de l'intégrateur de lumière est reliée directement à l'entrée (17) du coupleur de faisceau (11).

15. Dispositif selon l'une des revendications 1 à 14, **caractérisé en ce que** les capteurs (38) sont agencés de manière à détecter séparément le modèle spectral et/ou des données électro-physiologiques et/ou des données physiologico-chimiques.

16. Dispositif selon la revendication 15, **caractérisé en ce que** le capteur (38) comporte, pour la détermination du modèle spectral, des monochromateurs et/ou des filtres éventuellement commandables et/ou des analyseurs de spectres (58).

17. Dispositif selon la revendication 15 ou 16, **caractérisé en ce qu**'un convertisseur de données, qui est accordé sur les données à déterminer, est branché en aval de chacun des capteurs (38).
